# EUROPEAN PATENT APPLICATION

(11) **EP 4 059 558 A1**
(43) Date of publication of application: **21.09.2022**
(21) Application number: 20887118.6
(22) Date of filing: 11.11.2020
(51) Int. Cl.: A61M 37/00

(54) **VORTEXED-ACOUSTIC-FIELD METHOD FOR CREATING CELL PERMEABILITY AND VORTEXED-ACOUSTIC-FIELD METHOD FOR OPENING BLOOD-BRAIN BARRIER**

(30) Priority: 12.11.2019 US 201962934032 P
(71) Applicant: National Tsing Hua University, Hsinchu City 30013 (TW)
(72) Inventor: YEH, Chih-Kuang, Hsinchu City, Taiwan 30013 (TW); LO, Wei-Chen, Hsinchu City, Taiwan 30013 (TW); FAN, Ching-Hsiang, Hsinchu City, Taiwan 30013 (TW); LAI, Chun-Yen, Hsinchu City, Taiwan 30013 (TW)
(74) Representative: Kuhnen & Wacker Patent- und Rechtsanwaltsbüro PartG mbB
(86) International application number: PCT/CN2020/128061
(87) International publication number: WO 2021/093762

(57) **Abstract**

The present invention provides a vortexed-acoustic-field method for creating cell permeability and opening blood-brain barrier, which comprises using an ultrasonic transducer; using a probe emitting a vortexed acoustic field, the probe emitting a vortexed acoustic field having a piezoelectric patch comprising at least one inner channel, where when there is a plurality of inner channels, a phase difference is generated between each two channels, which is used by the ultrasonic transducer to generate a vortex in an acoustic channel; and generating a vortexed acoustic field to enable the cells to have permeability, where the vortexed acoustic field has the following parameters: frequency: 20 kHz to 20 MHz, and acoustic pressure: 0.1 to 10 MPa.

## Description

### BACKGROUND

### Technical Field

The present invention relates to the use of a vortex, and more particularly to a vortexed-acoustic-field method for creating cell permeability and opening a blood-brain barrier.

### Related Art

In the treatment of thrombus-related diseases, such as myocardial infarction, deep venous thrombosis, ischemic stroke, and others, the common thrombolytic therapy in clinic requires intravenous injection of a thrombolytic agent within a few hours after the onset of symptoms. The thrombolytic drugs include anticoagulants and thrombolytics. However, some hazards exist due to the different constitutions of the patients, and bleeding complications can occur when the dosage is too high.

Besides the thrombolytic agents, ultrasound is a common therapy in thrombolytic treatment, which uses a low-frequency ultrasound of 20 kHz to 2 MHz. The low-frequency ultrasound beams can penetrate a depth and then be focused, to reduce the therapeutic dose of a thrombolytic agent. In the field of ultrasonic emission technologies, generally the focusing of focused or non-focused ultrasound is created by phase modulation. In order to reduce the dose of the thrombolytic agent and the risk of bleeding and take into account the thrombolytic effect, an interventional catheter is used to deliver the thrombolytic agent directly to the site of thrombus. However, the use of a catheter to deliver the thrombolytic agent in combination with the general phase modulation to produce an ultrasound to enhance the thrombus ablation by the agent remains an invasive treatment and is a generally accepted prior art.

Moreover, with the aid of an ultrasonic contrast agent in the form of microbubbles, the therapeutic effect of thrombolysis can be promoted. However, the previous practice is to inject microbubbles intravenously, and then irradiate with a pulsed ultrasound after the microbubbles reach the blood vessels in the brain with the blood flow, upon which the microbubbles will absorb the energy of the ultrasonic waves to allow their volumes to periodically expand and contract. This process increases the cell membrane permeability of vascular endothelial cells or disrupts the tight junctions between the endothelial cells, resulting in the opening of the blood-brain barrier. Irradiation of ultrasound is non-invasive, and intravenous injection of microbubbles is invasive. For the use in glioma, Alzheimer's disease, and amyotrophic lateral sclerosis, this technology is still under clinical trials (clinical trials, gov; NCT02986932, NCT03119961, NCT03321487, NCT02343991, and NCT03551249).

Generally, common phase modulation produces an ultrasound to enhance the thrombus ablation by a drug to achieve an enhanced thrombolytic effect. However, the common phase modulation is different from the thrombolysis technology disclosed in the present invention that produces vortexed acoustic field by phase modulation. In view of this, the technical effect of the present invention adopting phase modulation to generate a vortexed-acoustic-field differs in that the common phase modulation and the phase modulation of the present invention that generates a vortexed-acoustic-field are two completely different technologies. The probe emitting a vortexed-acoustic-field in the present invention has a piezoelectric patch comprising at least one inner channel. When there is a plurality of inner channels, a phase difference is generated between each two channels, which is used by an ultrasonic transducer to generate a vortexed-acoustic-field. In short, although phase modulation is adopted in both technologies, the techniques are not the same. The thrombolysis effect can be improved, and the present invention can further be promoted by a combination of the phase modulation in the present invention to generate a vortexed-acoustic-field with a microbubble input device.

### SUMMARY

The problem to be solved by the present invention is to provide a vortexed-acoustic-field method for creating cell permeability and opening blood-brain barrier, and use thereof. In this method, only an ultrasound of vortexed acoustic field is irradiated, which is non-invasive, so as to improve the traditional invasive means in which a common phase-regulated ultrasound (US) is used to enhance the thrombus ablation by a drug. The main improvement in the present invention is that the opening of cell permeability and increased thrombolysis are achieved without intravenous injection of microbubbles. Another improvement in the present invention is that not only an ultrasound of vortexed acoustic field is irradiated, but also a microbubble input device can be used to facilitate the generation of microbubbles for treatment.

An object of the present invention is to provide a vortexed-acoustic-field method for creating cell permeability, which comprises using an ultrasonic transducer; using a probe emitting a vortexed acoustic field, the probe emitting a vortexed acoustic field having a piezoelectric patch comprising at least one inner channel, where when there is a plurality of inner channels, a phase difference is generated between each two channels, which is used by the ultrasonic transducer to generate a vortexed acoustic field; and enabling the cells to have permeability by the vortexed acoustic field, where the vortexed acoustic field has the following parameters: frequency: 20 kHz to 20 MHz, and acoustic pressure: 0.1 to 10 MPa.

According to the above object, when the following parameters: frequency: 0.5 MHz to 20 MHz, and acoustic pressure: 0.1 to 10 MPa, proving that the ultrasound of vortexed acoustic field of the present invention is effective and can greatly shorten the time to open the cell permeability.

According to the above object, the probe emitting a vortexed acoustic field of the present invention comprises at least one piezoelectric patch, or consists of an array of at least four piezoelectric patches, or consists of an array of at least three piezoelectric patches.

The probe emitting a vortexed acoustic field of the present invention comprises at least four piezoelectric patches, proving that the ultrasound of vortexed acoustic field of the present invention is more effective and can greatly shorten the time to open the cell permeability.

An object of the present invention is to provide a vortexed-acoustic-field method for creating cell permeability, which comprises using an ultrasonic transducer; using a radial probe emitting a vortexed acoustic field, the radial probe emitting a vortexed acoustic field being capable of performing vortex motion at both sides, and the radial probe emitting a vortexed acoustic field having a piezoelectric patch comprising at least one inner channel, where when there is a plurality of inner channels, a phase difference is generated between each two channels, which is used by the ultrasonic transducer to generate a vortexed acoustic field; and enabling the cells to have permeability by the vortexed acoustic field, where the vortexed acoustic field has the following parameters: frequency: 20 kHz to 20 MHz, and acoustic pressure: 0.1 to 10 MPa.

According to the above object, when the following parameters: frequency: 0.5 MHz to 20 MHz, and acoustic pressure: 0.1 to 10 MPa, proving that the ultrasound of vortexed acoustic field of the present invention is effective and can greatly shorten the time to open the cell permeability.

An object of the present invention is to provide a vortexed-acoustic-field method for opening a blood-brain barrier, which comprises: using an ultrasonic transducer; using a probe emitting a vortexed acoustic field, the probe emitting a vortexed acoustic field having a piezoelectric patch comprising at least one inner channel, where when there is a plurality of inner channels, a phase difference is generated between each two channels, which is used by the ultrasonic transducer to generate a vortexed acoustic field; and opening the blood-brain barrier by using the vortexed acoustic field, where the vortexed acoustic field has the following parameters: frequency: 20 kHz to 20 MHz, and acoustic pressure: 0.1 to 10 MPa.

According to the above object, when the following parameters: frequency: 0.5 MHz to 20 MHz, and acoustic pressure: 0.1 to 10 MPa, provide a vortexed-acoustic-field method for opening a blood-brain barrier.According to the above object, the probe emitting a vortexed acoustic field of the present invention comprises at least one piezoelectric patch, or consists of an array of at least four piezoelectric patches, or consists of an array of at least three piezoelectric patches.

The probe emitting a vortexed acoustic field of the present invention comprises at least four piezoelectric patches, provide a vortexed-acoustic-field method for opening a blood-brain barrier.

An object of the present invention is to provide a vortexed-acoustic-field method for opening a blood-brain barrier, which comprises: using an ultrasonic transducer; using a radial probe emitting a vortexed acoustic field, the radial probe emitting a vortexed acoustic field being capable of performing vortex motion at both sides and the radial probe emitting a vortexed acoustic field having a piezoelectric patch comprising at least one inner channel, where when there is a plurality of inner channels, a phase difference is generated between each two channels, which is used by the ultrasonic transducer to generate a vortexed acoustic field; and opening the blood-brain barrier by using the vortexed acoustic field, where the vortexed acoustic field has the following parameters: frequency: 20 kHz to 20 MHz, and acoustic pressure: 0.1 to 10 MPa.

According to the above object, when the following parameters: frequency: 0.5 MHz to 20 MHz, and acoustic pressure: 0.1 to 10 MPa, provide a vortexed-acoustic-field method for opening a blood-brain barrier.

An object of the present invention is to provide a vortexed-acoustic-field method for creating cell permeability by using an ultrasound and microbubbles in combination, which comprises: using a microbubble input device; using a radial probe emitting a vortexed acoustic field; and using a votex method for creating cell permeability, to generate a vortexed acoustic field; performing a focusing step to concentrate the microbubbles to a center of the vortexed acoustic field; and performing a manipulation step, to direct the bubbles delivered by a carrier to a site of lesion.

The piezoelectric patch used in the vortexed-acoustic-field method for creating cell permeability of the present invention has a radius of curvature in the range of 10 to 100 mm.

The ultrasound generation step of the present invention is performed by a pulse generator having a frequency in the range of 20 kHz to 20 MHz.

According to the above object, when the following parameters: frequency: 0.5 MHz to 20 MHz, and acoustic pressure: 0.1 to 10 MPa, proving that the ultrasound of vortexed acoustic field of the present invention is effective and can perform a focusing step to concentrate the microbubbles to a center of the vortexed acoustic field; and performing a manipulation step, to direct the bubbles delivered by a carrier to a site of lesion.The present invention has mainly the following effects. By opening the cell permeability and opening the blood-brain barrier, the substances that cannot pass through the blood-brain barrier are allowed to penetrate through the cerebral blood vessel into the brain tissue, which is a non-invasive treatment for the blood brain barrier and can improve the amount of a chemotherapeutic agent entering the brain tumor, so as to kill more tumor cells and improve the effect and use of the thrombolytic therapy. In addition, the present invention can be further facilitated by a combination of the vortexed acoustic field generated by phase modulation of an ultrasound with microbubbles, by which opening of cell permeability and increased rate of thrombus dissolution and use are achieved, proving that the ultrasound of vortexed acoustic field of the present invention is effective and can also greatly shorten the time to open the cell permeability.

For better understanding of the objects, functions, features and structures of the present invention, the present invention will be described below in connection with preferred embodiments with reference to accompanying drawings.

### BRIEF DESCRIPTION OF THE DRAWINGS

FIG. 1A is a schematic view of a vortex thrombus-dissolving system with an ultrasonic transducer according to an embodiment of the present invention;
FIG. 1B is a schematic view of a vortex thrombus-dissolving system with a piezoelectric patch according to an embodiment of the present invention;
FIG. 2 is a flow chart of a vortexed-acoustic-field method for creating cell permeability according to an embodiment of the present invention;
FIG. 3 is a flow chart of a vortexed-acoustic-field method for creating cell permeability according to another embodiment of the present invention;
FIG. 4 is a flow chart of a vortexed-acoustic-field method for opening a blood-brain barrier according to an embodiment of the present invention;
FIG. 5 is a flow chart of a vortexed-acoustic-field method for opening a blood-brain barrier according to another embodiment of the present invention;
FIG. 6 is a flow chart of a method for creating cell permeability by using an ultrasound and microbubbles in combination according to an embodiment of the present invention;
FIG. 7 is a schematic view showing the head of an ultrasound-irradiated mouse according to an embodiment of the present invention;
FIG. 8 is a schematic view showing the opening and closing of permeability according to the present invention;
FIG. 9 is a schematic view showing a vortexed acoustic field irradiating cells according to an embodiment of the present invention;
FIG. 10 is a schematic view showing a vortex generated after irradiating the cells with a vortexed acoustic field according to an embodiment of the present invention;
FIG. 11 is a view showing the staining result of a permeability test according to a preferred embodiment of the present invention; and
FIG. 12 is a view showing the staining results of permeability observed after different time after staining with propidium iodide.

### DETAILED DESCRIPTION

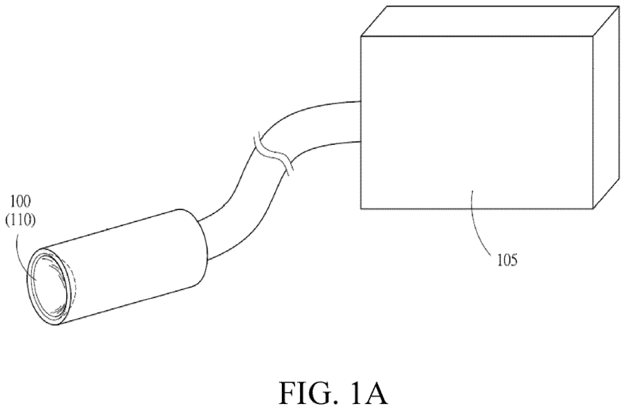

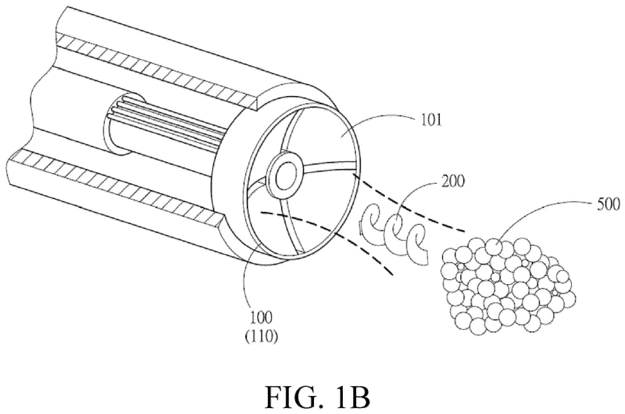

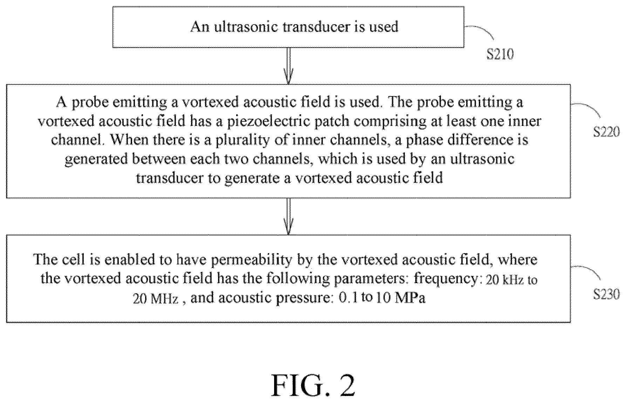

FIG. 1A is a schematic view of a thrombus-dissolving system with an ultrasonic transducer according to an embodiment of the present invention, FIG. 1B is a schematic view of a vortex thrombus-dissolving system with a piezoelectric patch according to an embodiment of the present invention, and FIG. 2 is a flow chart of a vortexed-acoustic-field method for creating cell permeability according to an embodiment of the present invention. Referring to FIGs 1A, 1B and 2, an embodiment of the present invention provides a vortexed-acoustic-field method for creating cell permeability, which comprises: using an ultrasonic transducer 105; using a probe 100 emitting a vortexed acoustic field, the probe 100 emitting a vortexed acoustic field having a piezoelectric patch 101 comprising at least one inner channel, where when there is a plurality of inner channels, a phase difference is generated between each two channels, which is used by the ultrasonic transducer 105 to generate a vortexed acoustic field 200; and enabling the cells to have permeability by the vortexed acoustic field 200, where the vortexed acoustic field 200 has the following parameters: frequency: 0.5 to 20 MHz, and acoustic pressure: 0.1 to 10 MPa.

The single-channel and multi-channel means of generating vortexed acoustic field can be respectively simply embodied as mechanical and electronic means. The familiar multi-channel means is to generate a vortexed acoustic field with at least 4 channels by individually driving a wave form with a phase difference of 2^{∗}pi/(channel number) or with time delay to each channel. In addition, the number of channels may be increased to produce a more delicate vortexed acoustic field, provided that the number of channels is an integer multiple of 4. In another embodiment, the multi-channel means is to generate a vortexed acoustic field with at least 3 channels by individually driving a wave form with a phase difference of 2^{∗}pi/(channel number) or with time delay to each channel.

Compared to the electronic means, the single-channel means of generating a vortexed acoustic field is as follows. An acoustic lens is added in front of a single-channel piezoelectric patch that has received geometric phase cut. When an ultrasound passes through the phase cut of the acoustic lens via the piezoelectric patch, a waveform of the vortexed acoustic field is produced. The difference between this means and the electronic means is mainly that a vortexed acoustic field can be generated by only a single-channel probe.

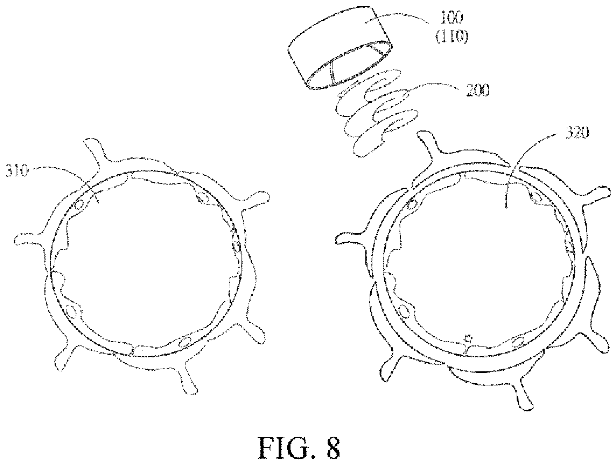

Referring to FIG. 8, Evans blue is used to assess the permeability to macromolecules.

In general, the cell permeability is closed 310, the serum albumin cannot cross the cell membrane barrier, and almost all Evans blue binds to albumin, so the dye cannot pass through the cell membrane barrier, and the nerve tissue is not stained. In contrast, when the cell permeability is opened 320, serum albumin will leak from the blood vessel into the tissue, the Evans blue bound to albumin can enter the cells, and the area where the dye is present will appear blue, that is, the cell permeability is opened 320.

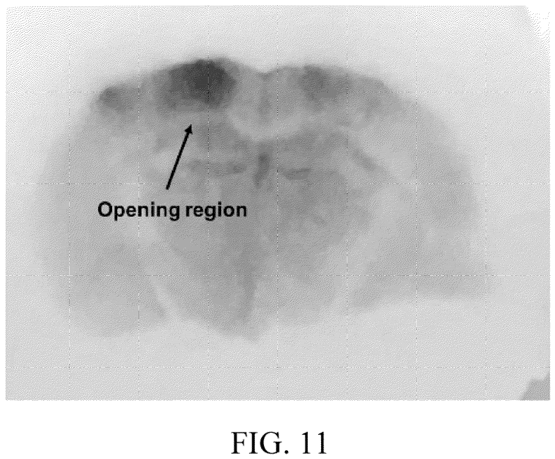

Referring to FIG. 11, the mice are intravenously injected with Evans blue. In an in-vivo mouse model in the head 300, in general, the cell permeability is closed 310. A vortexed acoustic field is applied. A probe 100 emitting a vortexed acoustic field is used. The probe 100 emitting a vortexed acoustic field has a piezoelectric patch 101 comprising at least one inner channel. When there is a plurality of inner channels, a phase difference is generated between each two channels, which is used by an ultrasonic transducer 105 to generate a vortexed acoustic field 200. It has been experimentally proved that after the ultrasound of vortexed acoustic field is applied (where the irradiation time is 10 minutes), the area where the dye is present appears blue, and the blue area in the figure can be observed by the naked eyes, indicating that the cell permeability is opened 320.

Referring to FIG. 2, in Step S210, an ultrasonic transducer is used.

Referring to FIG. 2, in Step S220, a probe emitting a vortexed acoustic field is used. The probe emitting a vortexed acoustic field has a piezoelectric patch comprising at least one inner channel. When there is a plurality of inner channels, a phase difference is generated between each two channels, which is used by an ultrasonic transducer to generate a vortexed acoustic field.

Referring to FIG. 2, in Step S230, the cell is enabled to have permeability by the vortexed acoustic field. The vortexed acoustic field has the following parameters: frequency: 0.5 to 20 MHz, and acoustic pressure: 0.1 to 10 MPa.

According to an embodiment of the present invention, the probe 100 emitting a vortexed acoustic field has at least one piezoelectric patch 101 or consists of an array of at least four piezoelectric patches 101. The piezoelectric patch 101 has a curved shape and is cut to have four adjacent channels, and a phase difference is present between two adjacent channels to generate an acoustic vortex.

In particular, the ultrasonic transducer 105 can be embodied as a pulse generator. More specifically, the ultrasonic transducer 105 can be, but is not limited to, a field programmable gate array (FPGA) pulse generator. In addition, the driving signal transmitted by the ultrasonic transducer 105 may be a square wave signal or a sine wave signal. Although not shown in the figures, an amplifier may be provided on the ultrasonic transducer 105 for amplification of the driving signal.

In an embodiment of the present invention, the piezoelectric patch 101 is made of a lead zirconate titanate material. Further, an epoxy resin is provided on the piezoelectric patch 101 and in a casing for filling and sealing the casing; however, the present invention is not limited thereto.

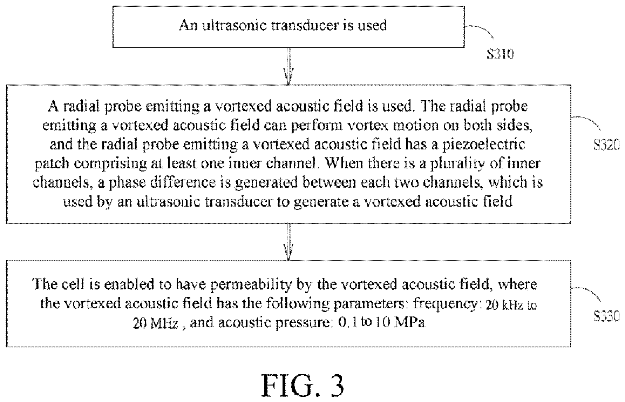

FIG. 3 is a flow chart of a vortexed-acoustic-field method for creating cell permeability according to another embodiment of the present invention. Referring to FIGs 1A, 1B and 3, an embodiment of the present invention provides a vortexed-acoustic-field method for creating cell permeability, which comprises: using an ultrasonic transducer 105; using a radial probe 110 emitting a vortexed acoustic field, the radial probe 110 emitting a vortexed acoustic field being capable of performing vortex motion at both sides and the radial probe 110 emitting a vortexed acoustic field having a piezoelectric patch 101 comprising at least one inner channel, where when there is a plurality of inner channels, a phase difference is generated between each two channels, which is used by the ultrasonic transducer 105 to generate a vortexed acoustic field 200; and enabling the cells to have permeability by the vortexed acoustic field 200, where the vortexed acoustic field 200 has the following parameters: frequency: 0.5 to 20 MHz, and acoustic pressure: 0.1 to 10 MPa.

According to an embodiment of the present invention, the radial probe 110 emitting a vortexed acoustic field has at least one piezoelectric patch 101 or consists of an array of at least four piezoelectric patches 101. The piezoelectric patch 101 has a curved shape and is cut to have four adjacent channels, and a phase difference is present between two adjacent channels to generate an acoustic vortex.

Referring to FIG. 3, in Step S310, an ultrasonic transducer is used.

Referring to FIG. 3, in Step S320, a radial probe emitting a vortexed acoustic field is used. The radial probe emitting a vortexed acoustic field can perform vortex motion at both sides, and the radial probe emitting a vortexed acoustic field has a piezoelectric patch comprising at least one inner channel. When there is a plurality of inner channels, a phase difference is generated between each two channels, which is used by an ultrasonic transducer to generate a vortexed acoustic field.

Referring to FIG. 3, in Step S330, the cell is enabled to have permeability by the vortexed acoustic field. The vortexed acoustic field has the following parameters: frequency: 0.5 to 20 MHz, and acoustic pressure: 0.1 to 10 MPa.

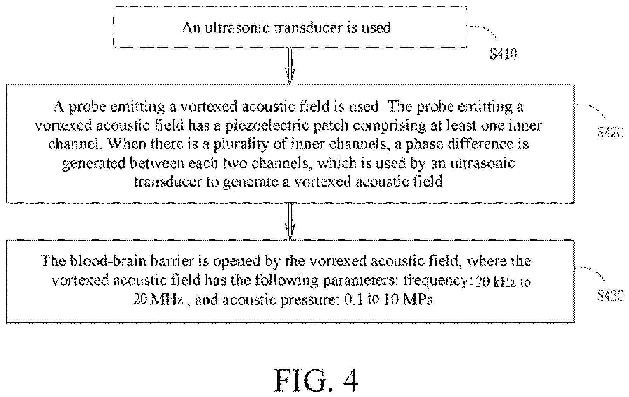

FIG. 4 is a flow chart of a vortexed-acoustic-field method for opening a blood-brain barrier according to an embodiment of the present invention. Referring to FIGs 1A, 1B and 4, an embodiment of the present invention provides a vortexed-acoustic-field method for opening a blood-brain barrier, which comprises: using an ultrasonic transducer 105; using a probe 100 emitting a vortexed acoustic field, the probe 100 emitting a vortexed acoustic field having a piezoelectric patch 101 comprising at least one inner channel, where when there is a plurality of inner channels, a phase difference is generated between each two channels, which is used by the ultrasonic transducer 105 to generate a vortexed acoustic field 200; and opening the blood-brain barrier by the vortexed acoustic field 200, where the vortexed acoustic field 200 has the following parameters: frequency: 0.5 to 20 MHz, and acoustic pressure: 0.1 to 10 MPa.

Blood-brain barrier (BBB) refers to a barrier between the blood vessels and the brain that selectively blocks certain substances from entering the brain. The important function of the blood-brain barrier is to prevent the brain from being affected by chemically conductive substances. Since many functions in the body are controlled by the brain by means of secretion of hormones, if chemically conductive substances are allowed to flow freely in the brain, feedback may occur. Therefore, if the brain function is maintained in a normal mode of operation, the presence of a blood-brain barrier will also prevent the brain from being infected by the bacteria.

Evans blue is used to assess the permeability of blood brain barrier to macromolecules. In general, the serum albumin cannot cross the barrier, and almost all Evans blue binds to albumin, so the dye cannot pass through the blood brain barrier, and the nerve tissue is not stained. In contrast, when the blood brain barrier is damaged or opened, the albumin will leak from the blood vessel into the peripheral brain tissue, the Evans blue bound to albumin can enter the central nervous system (CNS), and the area where the dye is present will appear blue.

Before sonication, the mice are intravenously injected with Evans blue. In an in-vivo mouse model in the head 300, the dye is not able to pass through the blood-brain barrier at this time. However, after the probe 100 emitting a vortexed acoustic field is used, the blood-brain barrier is opened. It will leak from the blood vessel to the peripheral brain tissue, and the area where the dye is present, that is, the area where the blood brain barrier is opened, will appear blue. In this way, the thrombus 500 in the human or animal can be further treated.

### A preferred example of the present invention

### Experimental method:

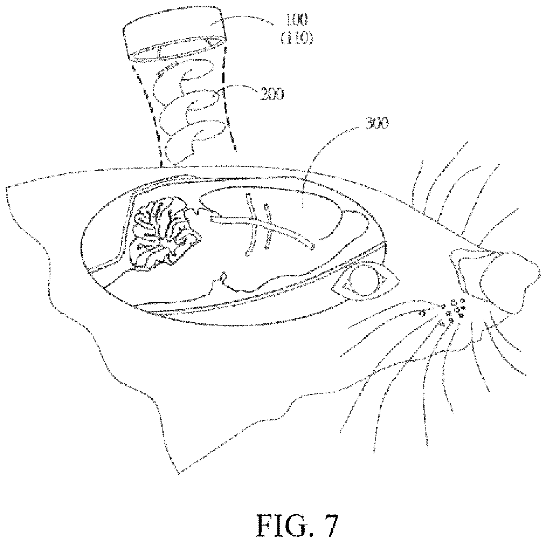

FIG. 7 is a schematic view showing the head of an ultrasound-irradiated mouse according to an embodiment of the present invention. In the in-vivo mouse model, the mice in the group teated with an ultrasound of vortexed acoustic field and the mice in the group treated with a common ultrasound were injected intravenously with Evans blue, and then sonicated.

Group treated with a common ultrasound: The mouse head 300 was applied with a conductive gel and sonicated with a common ultrasound (sonication time: 10 minutes).

Group treated with an ultrasound of vortexed acoustic field of the present invention: The mouse head 300 was applied with a conductive gel and irradiated with a probe 100 emitting a vortexed acoustic field of the present invention (sonication time: 10 minutes), where the probe emitting a vortexed acoustic field consisted of an array of four piezoelectric patches.

After sonication, the rat brain was removed, frozen, and sliced, and whether a blue area is present was visually observed. If a blue area is present, it is the area where the blood brain barrier is opened.

The ultrasound has the following parameters:
Frequency: 3.1 MHz, 7 MHz
Acoustic pressure: 1 MPa
Duty cycle: 50%
Time: 10 minutes
Experimental result:

FIG. 11 is a view showing the staining result of a permeability test according to a preferred embodiment of the present invention, particularly a view showing the staining result of a permeability test of a blood-brain barrier. The figure includes a left and a right side, the right side is the group treated with a common ultrasound, and the left side is the group treated with an ultrasound of vortexed acoustic field. Group treated with a common ultrasound: The mouse head 300 is applied with a conductive gel and sonicated with a common ultrasound (sonication time: 10 minutes). After sonication, the rat brain is removed, frozen, and sliced, and visually observed. The figure shows that only a small blue area is present, indicating that only a small portion of the blood-brain barrier is opened.

Group treated with an ultrasound of vortexed acoustic field of the present invention: The mouse head 300 is applied with a conductive gel and irradiated with a probe 100 emitting a vortexed acoustic field of the present invention (sonication time: 10 minutes). After sonication, the rat brain is removed, frozen, and sliced, and visually observed. The figure shows that several large blue areas are present, indicating that the blood-brain barrier is obviously opened. FIG. 8 is a schematic view showing the opening and closing of permeability according to the present invention.

According to an embodiment of the present invention, the probe 100 emitting a vortexed acoustic field has at least one piezoelectric patch 101 or consists of an array of at least four piezoelectric patches 101. The piezoelectric patch 101 has a curved shape and is cut to have four adjacent channels, and a phase difference is present between two adjacent channels to generate an acoustic vortex.

Referring to FIG. 4, in Step S410, an ultrasonic transducer is used.

Referring to FIG. 4, in Step S420, a probe emitting a vortexed acoustic field is used. The probe emitting a vortexed acoustic field has a piezoelectric patch comprising at least one inner channel. When there is a plurality of inner channels, a phase difference is generated between each two channels, which is used by an ultrasonic transducer to generate a vortexed acoustic field.

Referring to FIG. 4, in Step S430, the blood-brain barrier is opened by the vortexed acoustic field. The vortexed acoustic field has the following parameters: frequency: 0.5 to 20 MHz, and acoustic pressure: 0.1 to 10 MPa.

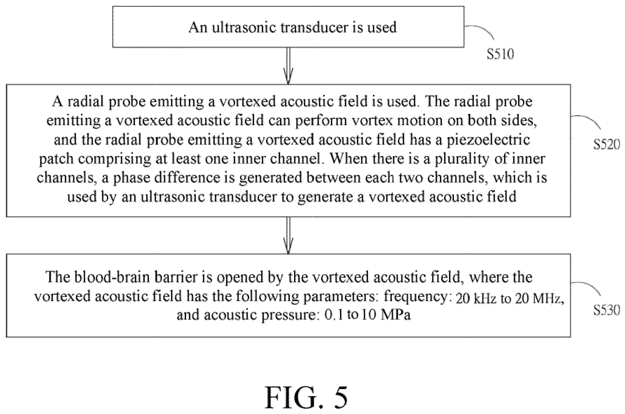

FIG. 5 is a flow chart of a vortexed-acoustic-field method for opening a blood-brain barrier according to another embodiment of the present invention. Referring to FIGs 1A, 1B and 5, an embodiment of the present invention provides a vortexed-acoustic-field method for opening a blood-brain barrier, which comprises: using an ultrasonic transducer 105; using a radial probe 110 emitting a vortexed acoustic field, the radial probe 110 emitting a vortexed acoustic field being capable of performing vortex motion at both sides and the radial probe 110 emitting a vortexed acoustic field having a piezoelectric patch 101 comprising at least one inner channel, where when there is a plurality of inner channels, a phase difference is generated between each two channels, which is used by the ultrasonic transducer 105 to generate a vortexed acoustic field 200; and opening the blood-brain barrier by the vortexed acoustic field 200, where the vortexed acoustic field 200 has the following parameters: frequency: 0.5 to 20 MHz, and acoustic pressure: 0.1 to 10 MPa.

Referring to FIG. 5, in Step S510, an ultrasonic transducer is used.

Referring to FIG. 5, in Step S520, a radial probe emitting a vortexed acoustic field is used. The radial probe emitting a vortexed acoustic field can perform vortex motion at both sides, and the radial probe emitting a vortexed acoustic field has a piezoelectric patch comprising at least one inner channel. When there is a plurality of inner channels, a phase difference is generated between each two channels, which is used by an ultrasonic transducer to generate a vortexed acoustic field.

Referring to FIG. 5, in Step S530, the blood-brain barrier is opened by the vortexed acoustic field. The vortexed acoustic field has the following parameters: frequency: 0.5 to 20 MHz, and acoustic pressure: 0.1 to 10 MPa.

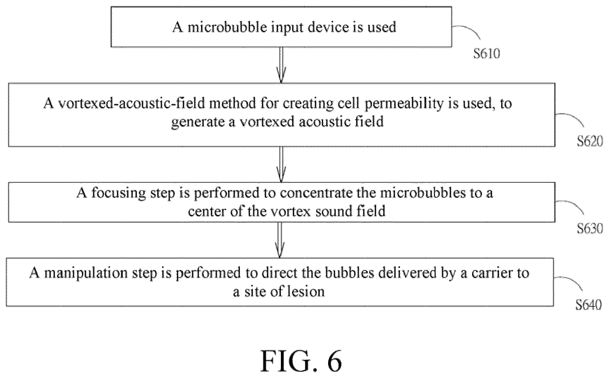

FIG. 6 is a flow chart of a method for creating cell permeability by using an ultrasound and microbubbles in combination according to an embodiment of the present invention. Referring to FIG. 6, an embodiment of the present invention provides a method for creating cell permeability by using an ultrasound and microbubbles in combination, which comprises: using a microbubble input device; using a radial probe emitting a vortexed acoustic field; and using a votex method for creating cell permeability, to generate a vortexed acoustic field; performing a focusing step to concentrate the microbubbles to a center of the vortexed acoustic field; and performing a manipulation step, to direct the bubbles delivered by a carrier to a site of lesion.

The common ultrasonic contrast agent is a micro-bubble. The ultrasonic contrast agent is a micro-bubble that can be encapsulated by a shell of a biodegradable material. It has the function of enhancing the ultrasonic scattering signal, and has the function of marking and guiding during ultrasonic imaging. When an ultrasound of sufficiently high acoustic pressure is applied to the microbubbles, the microbubbles will be broken. The ultrasonic microbubble contrast agent can be used in imaging and target drug delivery and to accelerate the dissolution of the thrombus 500.

Propidium iodide (PI) cannot penetrate the cell membrane under normal conditions, and the propidium iodide solution can enter cells that lose cell membrane permeability.

### A preferred example of the present invention

### Experimental method:

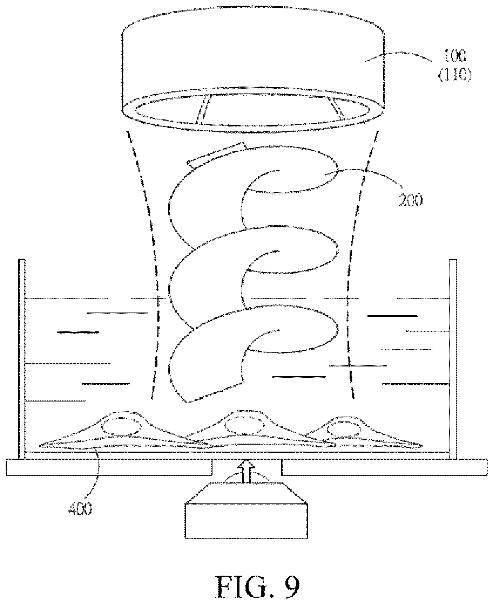

Referring to FIG. 9, no MBs (microbubbles) were added to the cell culture medium, and red blood cells were added to the culture medium to simulate the general blood environment. During the permeability test, a buffer that is a sterilized phosphate buffered saline (PBS) was mixed with red blood cells at a ratio of phosphate buffered saline:red blood cells = 1:1. A conductive glue was coated on the bottom of the cell 400 culture plate, and sonicated under different conditions. After staining with propidium iodide, the opening of permeability was observed at different times.

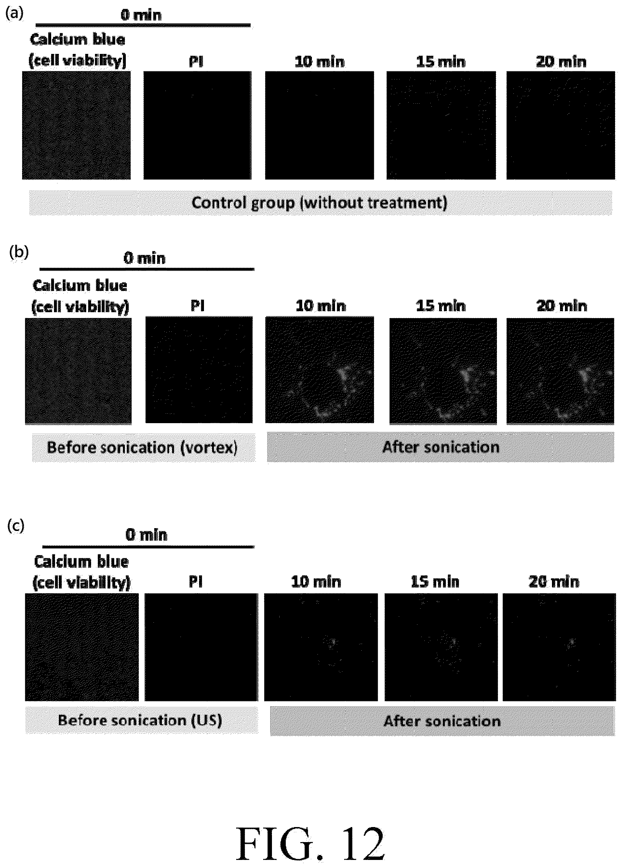

FIG. 12 is a view showing the staining results of permeability observed after different time after staining with propidium iodide. The cells were assayed for cell viability with Calcium blue under different experimental conditions, and the differences in permeability in different groups were compared by comparing the results of red fluorescence produced by the propidium iodide dye entering the cells in each group. (a) is the control group; (b) is the group treated with an ultrasound of vortexed acoustic field of the present invention; and (c) is the group treated with a common ultrasound. The cells are observed at different times. In the (b) group treated with an ultrasound of vortexed acoustic field of the present invention and the (c) group treated with a common ultrasound, the parameters are the same, including a frequency of 3 MHz, an acoustic pressure of 2.1 MPa, and a duty cycle of 1%.

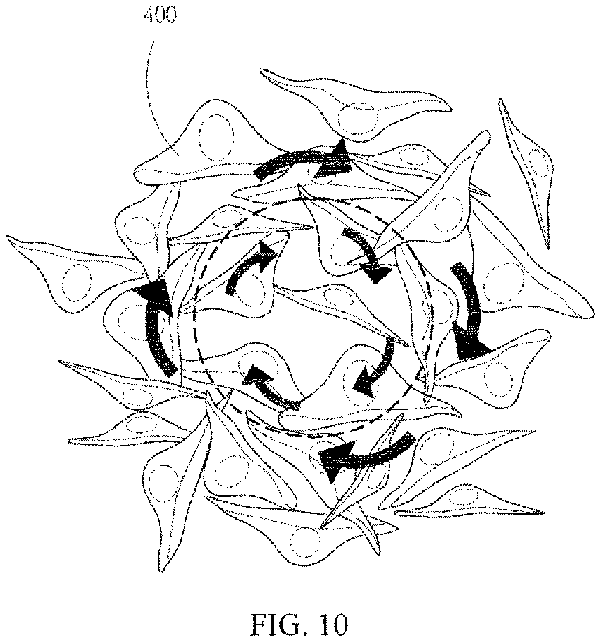

FIG. 9 is a schematic view showing the a vortexed acoustic field irradiating the cells according to an embodiment of the present invention. FIG. 10 is a schematic view showing the vortex generated after irradiating the cells with a vortexed acoustic field according to an embodiment of the present invention.

FIG. 12 is illustrated as follows:
FIG. 12(a) shows the cell survival detected by Calcium blue in the control group. The cells in this group are left untreated, and observed for the opening of permeability at different times including 10 minutes, 15 minutes, and 20 minutes, respectively after staining with propidium iodide.
FIG. 12(b) shows the cell survival detected by Calcium blue in the group treated with an ultrasound of vortexed acoustic field of the present invention. The cells in this group are treated with an ultrasound of vortexed acoustic field, and observed for the opening of permeability at different times including 10 minutes, 15 minutes, and 20 minutes, respectively after staining with propidium iodide.
FIG. 12(c) shows the cell survival detected by Calcium blue in the group treated with a common ultrasound. The cells in this group are treated with a common ultrasound, and observed for the opening of permeability at different times including 10 minutes, 15 minutes, and 20 minutes, respectively after staining with propidium iodide.

### Experimental result:

(a) is the control group; (b) is the group treated with an ultrasound of vortexed acoustic field of the present invention; and (c) is the group treated with a common ultrasound. The cells are observed at different times. After staining with propidium iodide, the opening of permeability was observed at different times in a cell experiment.

The results in FIG. 12 are described below.

FIG. 12(a) shows that in the control group, the cells are not observed to produce red fluorescence at different times, including 10 minutes, 15 minutes, and 20 minutes respectively after staining with propidium iodide, indicating that the permeability is still closed.

FIG. 12(b) shows that in the group treated with an ultrasound of vortexed acoustic field, the cells are observed to produce quantities of red fluorescence at different times, including 10 minutes, 15 minutes, and 20 minutes respectively after staining with propidium iodide, indicating that the permeability is opened.

FIG. 12(c) shows that in the group treated with a common ultrasound, the cells are observed to produce only a little red fluorescence at different times, including 10 minutes, 15 minutes, and 20 minutes respectively after staining with propidium iodide, indicating that the permeability is just slightly opened.

The observations at 10 minutes of the control group, the group treated with an ultrasound of 10 minutes of vortexed acoustic field, and the group treated with a common ultrasound of 10 minutes are compared. As can be known from the results of red fluorescence produced by the PI dye entering the cells in each group, the brightness in FIG. 12(b) of the group treated with an ultrasound of vortexed acoustic field is obviously increased than that in other groups, indicating that the cell permeability is more opened after the vortexed acoustic field acts on the cells. This may be attributed to the fact that the ultrasound of vortexed acoustic field transmits an angular momentum to the cell culture medium; and when the buffer and the red blood cells in the cell culture medium absorb the angular momentum, streaming occurs, which produces a strong shear stress after contacting the cell surface, thus resulting in an increase in cell permeability. This result can also interpret why the blood-brain barrier is more susceptible to permeability opening after being exposed to the ultrasound of vortexed acoustic field.

Moreover, the same method is used in an in-vivo mouse model to open the blood-brain barrier by a vortexed acoustic field. It has been confirmed that the effect of the vortexed acoustic field to open the cell permeability works well not only in in-vitro experiments, but also in in-vivo experiments.

The piezoelectric patch used in the vortexed-acoustic-field method for creating cell permeability according to an embodiment has a radius of curvature in the range of 10 to 100 mm.

In an embodiment of the present invention, the ultrasound generation step of the present invention is performed by a pulse generator having a frequency in the range of 0.5 to 20 MHz.

Referring to FIG. 6, in Step S610, a microbubble input device is used.

Referring to FIG. 6, in Step S620, a vortexed-acoustic-field method for creating cell permeability is used, to generate a vortexed acoustic field.

Referring to FIG. 6, in Step S630, a focusing step is performed to concentrate the microbubbles to a center of the vortexed sound field.

Referring to FIG. 6, in Step S640, a manipulation step is performed to direct the bubbles delivered by a carrier to a site of lesion.

Therefore, the present invention has excellent advancement and practicability in similar products. Moreover, after searching for domestic and foreign technical documents concerning such products, it is true that no identical or similar structure or technology exists before the present application. Therefore, the present invention meets the patent requirements of "Novelty", "industrial applicability" and "inventive steps", and applied in accordance with the law.

The above description is merely preferred embodiments of the present invention, and other equivalent structural changes of the present invention made in accordance with the disclosure and the scope of the invention are intended to be embraced in the scope of the present invention.

## Claims

1. A vortexed-acoustic-field method for creating cell permeability, comprising:
using an ultrasonic transducer;
using a probe emitting a vortexed acoustic field, the probe emitting a vortexed acoustic field having a piezoelectric patch comprising at least one inner channel, wherein when there is a plurality of inner channels, a phase difference is generated between each two channels, which is used by the ultrasonic transducer to generate a vortexed acoustic field; and
enabling the cells to have permeability by the vortexed acoustic field, wherein the vortexed acoustic field has the following parameters: frequency: 20 kHz to 20 MHz, and acoustic pressure: 0.1 to 10 MPa.

2. The vortexed-acoustic-field method for creating cell permeability according to claim 1, the following parameters: frequency: 0.5 MHz to 20 MHz, and acoustic pressure: 0.1 to 10 MPa.

3. The vortexed-acoustic-field method for creating cell permeability according to claim 1, wherein the probe emitting a vortexed acoustic field consists of an array of at least three piezoelectric patches.

4. The vortexed-acoustic-field method for creating cell permeability according to claim 1, wherein the probe emitting a vortexed acoustic field consists of an array of at least four piezoelectric patches.

5. A vortexed-acoustic-field method for creating cell permeability, comprising:
using an ultrasonic transducer;
using a radial probe emitting a vortexed acoustic field, the radial probe emitting a vortexed acoustic field being capable of performing vortex motion at both sides, and the radial probe emitting a vortexed acoustic field having a piezoelectric patch comprising at least one inner channel, wherein when there is a plurality of inner channels, a phase difference is generated between each two channels, which is used by an ultrasonic transducer to generate a vortexed acoustic field; and
enabling the cells to have permeability by the vortexed acoustic field, wherein the vortexed acoustic field has the following parameters: frequency: 20 kHz to 20 MHz, and acoustic pressure: 0.1 to 10 MPa.

6. The vortexed-acoustic-field method for opening a blood-brain barrier according to claim 5, the following parameters: frequency: 0.5 MHz to 20 MHz, and acoustic pressure: 0.1 to 10 MPa.

7. A vortexed-acoustic-field method for opening a blood-brain barrier, comprising:
using an ultrasonic transducer;
using a probe emitting a vortexed acoustic field, the probe emitting a vortexed acoustic field having a piezoelectric patch comprising at least one inner channel, wherein when there is a plurality of inner channels, a phase difference is generated between each two channels, which is used by the ultrasonic transducer to generate a vortexed acoustic field; and
opening the blood-brain barrier by the vortexed acoustic field, wherein the vortexed acoustic field has the following parameters: frequency: 20 kHz to 20 MHz, and acoustic pressure: 0.1 to 10 MPa.

8. The vortexed-acoustic-field method for opening a blood-brain barrier according to claim 7, the following parameters: frequency: 0.5 MHz to 20 MHz, and acoustic pressure: 0.1 to 10 MPa.

9. The vortexed-acoustic-field method for opening a blood-brain barrier according to claim 7, wherein the probe emitting a vortexed acoustic field consists of an array of at least three piezoelectric patches.

10. The vortexed-acoustic-field method for opening a blood-brain barrier according to claim 7, wherein the probe emitting a vortexed acoustic field consists of an array of at least four piezoelectric patches.

11. A vortexed-acoustic-field method for opening a blood-brain barrier, comprising:
using an ultrasonic transducer;
using a radial probe emitting a vortexed acoustic field, the radial probe emitting a vortexed acoustic field being capable of performing vortex motion at both sides, and the radial probe emitting a vortexed acoustic field having a piezoelectric patch comprising at least one inner channel, wherein when there is a plurality of inner channels, a phase difference is generated between each two channels, which is used by an ultrasonic transducer to generate a vortexed acoustic field; and
opening the blood-brain barrier by the vortexed acoustic field, wherein the vortexed acoustic field has the following parameters: frequency: 20 kHz to 20 MHz, and acoustic pressure: 0.1 to 10 MPa.

12. The vortexed-acoustic-field method for opening a blood-brain barrier according to claim 11, the following parameters: frequency: 0.5 MHz to 20 MHz, and acoustic pressure: 0.1 to 10 MPa.

13. A method for creating cell permeability by using ultrasound and microbubbles in combination, comprising:
using a microbubble input device;
using a vortexed-acoustic-field method for creating cell permeability according to claim 1 to generate a vortexed acoustic field;
performing a focusing step to concentrate the microbubbles to a center of the vortexed acoustic field; and
performing a manipulation step, to direct the bubbles delivered by a carrier to a site of lesion.

14. The method for creating cell permeability by using ultrasound and microbubbles in combination according to claim 13, wherein the piezoelectric patch used in the vortexed-acoustic-field method for creating cell permeability has a radius of curvature in the range of 10 to 100 mm.

15. The method for creating cell permeability by using ultrasound and microbubbles in combination according to claim 14, wherein the ultrasound generation step is performed by a pulse generator having a frequency in the range of 20 kHz to 20 MHz.

16. The method for creating cell permeability by using ultrasound and microbubbles in combination according to claim 15, the following parameters: frequency: 0.5 MHz to 20 MHz.
